# EUROPÄISCHE PATENTSCHRIFT

(11) **EP 3 514 134 B1**
(45) Veröffentlichungstag und Bekanntmachung des Hinweises auf die Patenterteilung: **18.08.2021**
(21) Anmeldenummer: 19152386.9
(22) Anmeldetag: 17.01.2019
(51) Int. Cl.: C07C 41/56, C07C 41/58, C07C 43/30

(54) **VERFAHREN ZUR TRENNUNG VON OXYMETHYLENETHERN**
PROCESS FOR THE SEPARATION OF OXYMETHYLENE ETHERS
PROCÉDÉ POUR LA SÉPARATION DES OXYMÉTHYLÈNE ÉTHERS

(30) Priorität: 19.01.2018 DE 102018101216
(43) Veröffentlichungstag der Anmeldung: 24.07.2019
(73) Patentinhaber: Rheinisch-Westfälische Technische Hochschule (RWTH) Aachen, 52056 Aachen (DE)
(72) Erfinder: Palkovits, Regina, 52074 Aachen (DE); Delidovich, Irina, 52074 Aachen (DE); Gierlich, Christian Henning, 52074 Aachen (DE)
(74) Vertreter: Michalski Hüttermann & Partner Patentanwälte mbB

(56) Entgegenhaltungen:
- EP-A1- 2 810 930
- EP-A1- 2 853 524
- DE-A1-102005 027 702
- US-A1- 2015 291 722

## Beschreibung

Die Erfindung betrifft ein Verfahren zur Trennung von Oxymethylenethern aus Gemischen, insbesondere ein Verfahren zur Abtrennung von Methylal und OME₂ von Wasser.

Als Oxymethylenether (OME) werden Verbindungen der chemischen Struktur H₃C-O-(CH₂O)ₙ-CH₃ bezeichnet. Das kleinste Oligomer mit n = 1 wird gemäß IUPAC als Dimethoxymethan, üblicherweise als Methylal oder kurz als OME₁ bezeichnet. Oligomere Verbindungen mit n ≥ 2 werden als Polyoxymethylendimethylether bezeichnet. Oxymethylenether werden derzeit als Diesel-Zusatzstoffe bzw. sogar langfristig als Alternative zu Dieselkraftstoff diskutiert. Als Zugabe zu Dieselkraftstoff ermöglichen Oxymethylenether eine deutliche Schadstoffreduzierung von NOₓ und Ruß. Oxymethylenether mit n = 1 bis 5 lassen sich in beliebigen Mengen mit Diesel mischen, wobei insbesondere Oxymethylenether mit n = 3 bis 5 als Komponenten von Dieselkraftstoff oder vollständige Alternative zum Dieselkraftstoff in der Diskussion stehen.

Die Produktion für solche Zusatzstoffe in Kraftstoffen erfordert Anlagen im großen Maßstab, um den Markt ausreichend zu bedienen. Für solche Anlagen ist neben einer effektiven Reaktion auch die nachfolgende Aufreinigung von enormer Wichtigkeit, um einen effektiven Gesamtprozess zu gestalten. Bekannte Verfahren zur Herstellung von Oxymethylenethern beziehen sich zumeist auf die Herstellung von Methylal aus Methanol und einer Formaldehydquelle als Edukt und dessen Abtrennung aus dem Reaktionsgemisch. Die Aufarbeitung des Reaktionsgemisches erfolgt destillativ. So beschreibt die DE 3147320 A1 ein Verfahren zur Gewinnung von reinem Methylal durch Trennung von Methanol-Methylal-Gemischen über zwei Rektifizierkolonnen, die bei verschiedenen Drücken betrieben werden, wobei das Destillat der ersten Rektifizierkolonne in der zweiten Rektifizierkolonne unter höherem Druck weiter getrennt wird. Auch die EP 2 637 992 A1 beschreibt ein kontinuierliches Verfahren zur Herstellung und Abtrennung von Methylal durch Umsetzen einer Mischung enthaltend Formaldehyd und Methanol, umfassend das Einspeisen der Produktmischung in eine erste Destillationskolonne und Auftrennen in drei verschiedene Produktströme, wobei der Produktstrom vom Kopf der ersten Kolonne optional in eine weitere, bei höherem Druck betriebene, Rektifikationskolonne eingespeist wird. Weitere Dokumente des Standes der Technik, in denen die Abtrennung rein destillativ erfolgt, sind beispielsweise DE 10 2005 027702 A1, US 2015/291722 A1, EP 2 853 524 A1 und EP 2 810 930 A1. Die destillativen Verfahren zur Abtrennung von Methylal sind jedoch äußerst energieintensiv und aufwändig.

Die Synthese von Methylal aus Methanol und einer Formaldehydquelle als Edukt ermöglicht es auch die höheren Oxymethylenether wie OME₃₋₅ mittels dieses Verfahrens herzustellen, wie von Oestreich et al. 2017 in Chem. Eng. Sci., 163, 92-104 "Reaction kinetics and equilibrium parameters for the production of oxymethylene dimethyl ethers (OME) from methanol and formaldehyde" gezeigt wurde. Jedoch bleibt auch bei der Produktion langkettiger Oxymethylenether die nachfolgende Auftrennung eine Herausforderungen, beispielsweise die Auftrennung des Produktgemisches aus Oxymethylenethern verschiedener Kettenlängen. Mittels Destillation können die Oxymethylenether OME₃₋₅ aus dem Reaktionsgemisch entfernt werden, allerdings bleiben kleinen Oxymethylenether OME₁/OME₂ und Wasser als Gemisch übrig. Insbesondere die Abtrennung von OME₂ von Wasser über destillative Verfahren ist nicht nur energieintensiv, sondern auch schwierig, da OME₂ und Wasser beide bei 100 °C bzw. 102 °C sieden und das azeotrope Gemisch daher destillativ nicht trennbar ist.

Daher besteht ein Bedarf an alternativen Verfahren zur Trennung von Oxymethylenethern. Der vorliegenden Erfindung lag daher die Aufgabe zu Grunde, ein Verfahren zur Trennung von Oxymethylenethern zur Verfügung zu stellen, dass mindestens einen der vorgenannten Nachteile des Standes der Technik überwindet.

Diese Aufgabe wird gelöst durch ein Verfahren zur Trennung von Oxymethylenethern aus einem diese enthaltenden Gemisch, wobei man die Oxymethylenether durch Adsorption an Adsorbentien ausgewählt aus der Gruppe umfassend Aktivkohle und/oder hypervernetzte Polymere abtrennt.

Überraschend wurde gefunden, dass Oxymethylenether an Aktivkohle und hypervernetzte Polymere (HCPs) adsorbiert werden und somit aus einem Gemisch entfernt werden können. Eine Trennung mittels Adsorption hat insbesondere den Vorteil energetisch günstig zu sein. Eine energiearme Trennung ist industriell unter dem zunehmend wichtigen Gesichtspunkt der Energieeinsparungen ein wesentlicher Vorteil. Geeignete Adsorbentien sind kommerziell erhältlich und ermöglichen eine Abtrennung bei niedrigen Temperaturen und unter deutlich geringerem apparativem Aufwand.

Unter dem Begriff "Oxymethylenether" (OME) werden im Sinne dieser Erfindung Verbindungen der chemischen Struktur H₃C-O-(CH₂O)ₙ-CH₃ verstanden, wobei vorzugsweise n bei 1 bis 10 liegt. Das kleinste Oligomer mit n = 1 wird gemäß IUPAC als Dimethoxymethan, üblicherweise als Methylal oder kurz als OME₁ bezeichnet. Die Begriffe werden im Folgenden synonym verwendet.

Das Gemisch enthält in bevorzugten Ausführungsformen Wasser, wobei das Gemisch gegebenenfalls noch weitere Stoffe enthalten kann. Gemische, die bei der Herstellung von Oxymethylenethern durch Umsetzen von Methanol und Formaldehyd oder Formaldehyd-Vorläufern entstehen, enthalten nicht umgesetztes Formaldehyd und Methanol, sowie oligomere Oxymethylenether verschiedener Kettenlänge und Wasser. Bei der Adsorption nimmt das Adsorbens die Oxymethylenether selektiv auf, andere Bestandteile, insbesondere Wasser, verbleiben im Gemisch. Die adsorbierten Oxymethylenether können anschließend wieder desorbiert werden. Das Verfahren ist für die Trennung der unterschiedlichen Oxymethylenether von Wasser verwendbar, und im speziellen für die Trennung von 0ME₁ und/oder OME₂ aus Wasser via Adsorption vorteilhaft. Das Verfahren ist hierbei insbesondere für eine Abtrennung von Methylal und/oder OME₂, insbesondere von OME₂, von Wasser verwendbar. Die Trennung von OME₂ und Wasser stellt einen großen Vorteil des Verfahrens dar. OME₂ und Wasser sieden beide bei 100 °C bzw. 102 °C und sind daher nicht destillativ trennbar. Die Adsorption hingegen ist unabhängig von den Siedepunkten der einzelnen Komponenten des Gemisches.

Grundsätzlich adsorbieren alle Oxymethylenether an Aktivkohle oder hypervernetzte Polymere. Die Oxymethylenether können ausgewählt sein aus der Gruppe umfassend Dimethoxymethan (Methylal, OME₁), 2,4,6-Trioxaheptan (OME₂), 2,4,6,8-Tetraoxanonan (OME₃), 2,4,6,8,10-Pentaoxaundekan (OME₄), 2,4,6,8,10,12-Hexaoxatridekan (OME₅), 2,4,6,8,10,12,14-Heptaoxapentadekan (OME₆), 2,4,6,8,10,12,14,16-Octaoxaheptadekan (OME₇) und/oder 2,4,6,8,10,12,14,16,18-Nonaoxanonadekan (OME₈). Oxymethylenether ab OME₃ weisen Siedepunkte auf, die von Wasser ausreichend verschieden sind, und sind von Wasser und Methanol leicht über eine Destillation zu trennen. Für das Verfahren sind die Oxymethylenether daher vorzugsweise ausgewählt aus der Gruppe umfassend Methylal (OME₁) und/oder 2,4,6-Trioxaheptan (OME₂). Das Verfahren ist insbesondere für die Trennung von Methylal (OME₁) und/oder 2,4,6-Trioxaheptan (OME₂) von Wasser vorteilhaft verwendbar.

Das Verfahren mittels adsorptiver Trennung ist insbesondere energetisch günstig, da mehrschrittige energieintensive Destillationen bei hohen Temperaturen entfallen können. Demgegenüber kann die Temperatur bei einer Adsorption in weiten Bereichen variieren. In Ausführungsformen des Verfahrens kann man die Adsorption bei einer Temperatur im Bereich von ≥ 0 °C bis ≤ 120 °C, bevorzugt im Bereich von ≥ 10°C bis ≤ 90°C, vorzugsweise bei einer Temperatur im Bereich von ≥ 20°C bis ≤ 40°C durchführen. Insbesondere Adsoptionen bei Umgebungstemperatur oder leicht erhöhten Temperaturen sind energetisch günstig und damit wirtschaftlich. In Ausführungsformen kann eine nachfolgende Desorption bei einer Temperatur im Bereich von ≥ 0 °C bis ≤ 250 °C, bevorzugt im Bereich von ≥ 20°C bis ≤ 200°C, vorzugsweise bei einer Temperatur im Bereich von ≥ 60°C bis ≤ 80°C, durchgeführt werden. Insbesondere bei der Desorption kann die Temperatur zur Steuerung der Geschwindigkeit der Trennung genutzt werden. So kann eine Erhöhung der Temperatur zu einem schnelleren Ablauf, und damit bei technischen Prozessen zu wertvoller Zeitersparnis, führen.

Die Trennung mittels Adsorption hat nicht nur den Vorteil energetisch günstig zu sein, sondern auch, dass bei der Desorption ein Lösungsmittel gewählt werden kann, das entweder inert ist oder im Rahmen der Reaktion bzw. des Herstellungsverfahrens, in welchem die Trennung verwendet wird, als Edukt dient. Organische Lösungsmittel, insbesondere wasserfrei, sind bevorzugt. Das Lösungsmittel, mittels dessen man die nachfolgende Desorption durchführt, kann ausgewählt sein aus der Gruppe umfassend Alkohole, Ether, Aldehyde, Ketone und/oder deren Mischungen. Insbesondere Alkohole wie Methanol oder Ethanol sowie Aldehyde wie Aceton oder Ether wie Tetrahydrofuran sind geeignet. Ein bevorzugtes Lösungsmittel ist Methanol. Auch Mischungen von Methanol mit weiteren Alkoholen, Ether, Aldehyden oder Ketonen sind bevorzugt verwendbar. Methanol eignet sich besonders gut im Rahmen von Herstellungsverfahren für Oxymethylenether, wo es als Edukt eingesetzt wird und mit den eluierten Oxymethylenethern, insbesondere Methylal und OME₂, der Reaktion wieder zugeführt werden kann, wodurch aus Prozesssicht der weitere Trennaufwand reduziert wird. Dies stellt eine einfache Lösung zur Verfügung, den Herstellungsprozess zu verschalten, um energetisch auf einem möglichst günstigen Niveau zu arbeiten.

Auch ist mittels des Verfahrens reines OME₂ wasserfrei herstellbar. Beispielsweise können Methylal und OME₂ gemeinsam durch Adsorption von Wasser getrennt werden und anschließend mittels eines Desorptionsmittel mit einem Siedepunkt über 120 °C eluiert werden, wobei in einer anschließenden Destillation Methylal und OME₂ leicht getrennt werden können.

Die Adsorption basiert auf Wechselwirkung zwischen Adsorbat und Adsorbens und kann daher vor allem zur Trennung von Komponenten unterschiedlicher Polaritäten verwendet werden. Für das Verfahren eignen sich insbesondere Materialien, die zumindest teilweise unpolar sind wie Aktivkohle und hypervernetzte Polymere.

Der Begriff "hypervernetzte Polymere" (hyper cross-linked polymers (HCPs)) bezeichnet im Sinne der Erfindung eine Klasse von nanoporösen Materialien, insbesondere vernetzte Polystyrole oder sogenannte Davankov-Harze. Hypervernetzte Polymere sind basierend auf Friedel-Crafts-Chemie oder aus der Nachvernetzung von Vorläufern vom Polystyrol-Typ oder aus der Kondensation kleiner Bausteinen herstellbar. Hypervernetzte Polymere weisen eine unpolare oder, abhängig von Seitengruppen, zumindest teilweise unpolare Oberfläche auf. Hypervernetzte Polymere weisen zudem in vorteilhafter Weise eine große spezifische Oberfläche auf.

Bevorzugt verwendbare hypervernetzte Polymere sind erhältlich durch Friedel-Crafts-Alkylierung von Verbindungen der allgemeinen Formel (I),

ClCH₂-(R)ₙAr-CH₂Cl (I)

, wobei
Ar für eine Phenyl-, Naphthyl-, Biphenyl-, Bisphenyl- oder Methylenbiphenylgruppe oder eine Mischung aus mindestens zwei dieser Gruppen, insbesondere eine Phenyl- oder Biphenylgruppe oder deren Mischungen,
R für eine Alkyl-, Carboxyl-, Sulfonyl-, Nitro-, Amino- oder Hydroxygruppe oder eine Mischung aus mindestens zwei dieser Gruppen, insbesondere eine Alkyl-, Sulfonyl- oder Carboxylgruppe oder deren Mischungen, und
n für eine Zahl von 0 bis 4, insbesondere 1 oder 2, steht,
oder
durch Umsetzung einer Verbindung Rₙ(Ar), in der R, n und Ar die für die allgemeine Formel (I) angegebene Bedeutung haben, mit Dimethoxymethan und/oder Formaldehyd.

Falls mindestens ein R in den Verbindungen der allgemeinen Formel (I) eine Alkylgruppe ist, weist diese vorzugsweise 1 bis 10, insbesondere 1 bis 3 C-Atome auf. Die Alkylgruppe kann linear oder verzweigtkettig sein. Methyl- und Ethylgruppen sind besonders bevorzugt. Falls mindestens ein R in den Verbindungen der allgemeinen Formel (I) eine Carboxyl- oder Sulfonylgruppe ist, weist sie ein übliches Gegenkation, vorzugsweise ausgewählt aus H⁺, den Alkaliionen wie Na⁺, Li⁺, K⁺, Ammoniumionen R¹R²R³R⁴N⁺, in denen R¹, R², R³ und R⁴ unabhängig voneinander ausgewählt werden aus H, Alkylgruppen mit 1 bis 3 C-Atomen und Hydroxyalkylgruppen mit 2 bis 3 C-Atomen, auf.

Die Bildung der hypervernetzten Polymere aus den Verbindungen der allgemeinen Formel (I) kann durch Katalyse mittels Lewis-Säuren, wie FeCl₃, AlCl₃ oder SnCl₄, oder mittels Brønsted-Säuren wie H₂SO₄ oder CF₃SO₃H erfolgen. Alternativ können solche hypervernetzten Polymere durch Umsetzung eines Aromaten Rₙ(Ar), wobei R, n und Ar die für die allgemeine Formel (I) angegebene Bedeutung haben, mit einem externen Verzweigungsagens wie Dimethoxymethan oder Formaldehyd, hergestellt werden. Vorzugsweise wird die Herstellung unter Einstellung eines stoffmengenbezogenen Monomer/Initiator-Verhältnisses in einem Bereich von 1:1 bis 1:10; genauer 1:2 bis 1:4 und unter Wahl eines aprotischen Lösemittels wie etwa Dichlorethan, Dichlormethan oder Diethlyether und einem Konzentrationsbereich von 0,1-1 mol/L so geführt, dass sich eine Oberfläche (nach BET) des Polymers im Bereich von 100 m²/g bis 5000 m²/g, insbesondere von 400 m²/g bis 2000 m²/g ergibt; solche Polymere werden bevorzugt eingesetzt. Bevorzugte Polymere weisen eine nachstehend allgemein für einen Ausgangsbaustein mit n = 0 und Ar = Phenyl dargestellte Gerüststruktur der Formel (II) auf:

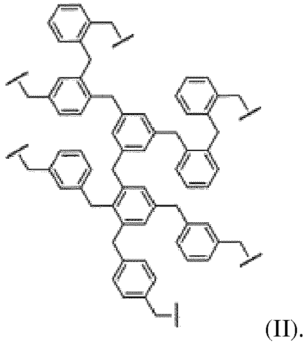

Bevorzugte hypervernetzte Polymere sind ausgebildet aus Monomeren ausgewählt aus der Gruppe umfassend 4,4-bis(chloromethyl)-1,10-biphenyl (BCMBP), 1,2-Dichloroxylen (o-DCX), 1,3-Dichloroxylen (m-DCX), 1,4-Dichloroxylen (p-DCX), 2,5-Dichloro-p-xylen (2,5-p-DCX), Triphenylamin (TPA), Dibenzofuran (DBF), Dibenzothiophen (DBT), Triphenylbenzol (TPB) und/oder Fluoren (FLUO). Hierbei können 4,4-bis(chloromethyl)-1,10-biphenyl (BCMBP), 1,2-Dichloroxylen (o-DCX), 1,3-Dichloroxylen (m-DCX), 1,4-Dichloroxylen (p-DCX) und 2,5-Dichloro-p-xylen (2,5-p-DCX) mit sich selbst polymerisiert werden, da sie Halogenidgruppen beinhalten, sowie als Verbinder (Linker) zur Polymerisation für Verbindungen wie TPA, TPB, DBT, DBF und FLUO dienen.

Ein bevorzugtes hypervernetztes Polymer ist hypervernetztes 4,4-Bis(chloromethyl)-1,10-biphenyl. Dieses kann beispielsweise eine Struktur gemäß der folgenden Formel (III) aufweisen:

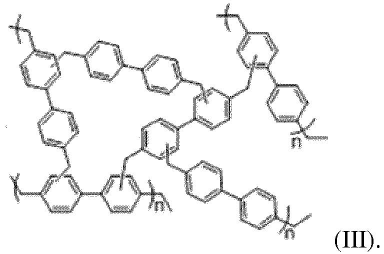

Es wurde festgestellt, dass die Adsorptionskapazität hypervernetzter Polymere bezüglich der Oxymethylenether etwa doppelt so hoch war wie bei Aktivkohlen. Weiterhin wurde festgestellt, dass die Wechselwirkung zwischen Oxymethylenethern und hypervernetzten Polymeren gering ist, sodass es nicht viel Energie bedarf um sie zu desorbieren. Die Adsorption an hypervernetzte Polymere stellt eine energiearme und einfache Möglichkeit dar die Trennung von Wasser und Oxymethylenethern durchzuführen. Hypervernetzte Polymere sind aus diesem Grund als Adsorbens besonders bevorzugt.

Unter dem Begriff "Aktivkohle" wird ein poröses, kohlenstoffhaltiges Produkt verstanden, das in der Lage ist, eine beträchtliche Anzahl von Verbindungen in sich aufzunehmen und an einer sehr großen Oberfläche anzulagern. Die spezifische Oberfläche herkömmlicher Aktivkohlen liegt zwischen 600 und 1,500 m²/g, es sind jedoch Aktivkohlen mit größerer Oberfläche erhältlich. Aktivkohle ist kommerziell erhältlich. Bevorzugt sind sphärische Aktivkohlen. Ebenfalls bevorzugt verwendbar sind extrudierte und pulverförmige Aktivkohlen.

In Ausführungsformen kann die spezifische Oberfläche der Aktivkohle im Bereich von ≥ 600 m²/g bis ≤ 3000 m²/g, im Bereich von ≥ 600 m²/g bis ≤ 1,500 m²/g, oder im Bereich von ≥ 600 m²/g bis ≤ 1000 m²/g liegen, wobei die spezifische Oberfläche nach dem BET-Verfahren bestimmt wird. Die spezifischen Oberfläche von Feststoffen kann durch Gasadsorption nach dem Brunauer-Emmett-Teller (BET)-Verfahren mittels der Adsorption von Stickstoff bestimmt werden. Die spezifische Oberfläche der hypervernetzte Polymere kann im Bereich von ≥ 800 m²/g bis ≤ 3000 m²/g liegen, vorzugsweise im Bereich von 1200 m²/g bis 3000 m²/g oder im Bereich von ≤ 1600 m²/g bis 3000 m²/g. Vorteilhaft sind große Oberflächen, wobei praktisch häufig ein Kompromiss zwischen möglichst hoher spezifische Oberfläche und einer gut handhabbaren Formgebung und mechanischen Stabilität des Adsorbens getroffen wird. So liegen hypervernetzte Polymere mit hoher spezifischer Oberfläche zumeist als Pulver vor, wobei feine Pulver verklumpen können und insbesondere industriell in großen Kolonnen schwierig zu handhaben sind. Eine Formgebung erfordert Bindemittel oder Zuschlagsstoffe. Insbesondere hypervernetzte Polymere können daher in geträgerter Form oder in Kugelform Verwendung finden, wodurch die Handhabbarkeit erleichtert wird. Insbesondere Aktivkohle ist in partikulärer Form kommerziell in guter Auswahl verfügbar. Auch extrudierte und pulverförmige Aktivkohle ist kommerziell in guter Auswahl verfügbar.

In bevorzugten Ausführungsformen verwendet man Aktivkohle oder hypervernetzte Polymere in Form sphärischer Partikel oder als Pulver. Die sphärischen Partikel weisen vorzugsweise einen mittleren Durchmesser im Bereich von ≥ 0,005 mm bis ≤ 200 mm, bevorzugt im Bereich von ≥ 0,05 mm bis ≤ 20 mm, auf. Unter dem Begriff "mittlerer Durchmesser" wird der Durchschnittswert aller Durchmesser oder der arithmetisch gemittelte Durchmesser bezogen auf die jeweiligen Partikel verstanden.

In weiteren Ausführungsformen sind insbesondere die hypervernetzte Polymere auch in Form von Partikeln mit einem mittleren Durchmesser im Bereich von ≥ 0,005 mm bis ≤ 200 mm, bevorzugt im Bereich von ≥ 0,05 mm bis ≤ 20 mm, verwendbar, wobei die Partikel unregelmäßig geformt sein können. Die Polymerpartikel können in Form eines Granulats oder granulatartig ausgebildet sein. In vorteilhafter Weise sind die hypervernetzten Polymere, insbesondere granulatartig ausgebildete Polymere, mehrfach in Adsorptions- und Desorptionszyklen verwendbar.

Insgesamt kann ein Verfahren zur Trennung von Oxymethylenethern aus einem diese enthaltenden Gemisch zur Verfügung gestellt werden, das insbesondere eine Trennung von Methylal und OME₂ von Wasser erlaubt. Die adsorptive Trennung der Oxymethylenether an Aktivkohle oder hypervernetzte Polymere ist energetisch günstiger als bekannte destillative Verfahren. Das Verfahren hat den zusätzlichen Vorteil, dass bei der Desorption Methanol als Lösungsmittel verwendet werden kann, welches im Rahmen der Synthese der Oxymethylenether als Edukt verwendet wird. Das Verfahren ist in vorteilhafter Weise im Rahmen von Herstellungsprozessen für Oxymethylenether verwendbar. So kann das Trennungsverfahren in Herstellungsprozesse für Oxymethylenether integriert werden, welche hierdurch auf einem besonders günstigen energetischen Niveau arbeiten können.

Ein weiterer Aspekt der Erfindung betrifft ein kontinuierliches Verfahren zur Herstellung und Abtrennung von Oxymethylenethern durch Umsetzen einer Reaktionsmischung enthaltend Formaldehyd und Methanol, wobei man das gebildete Rohprodukt destillativ in verschiedene Produktströme trennt, wobei ein erster Produktstrom eine Mischung an Oxymethylendimethylethern OME₃₋₅ umfasst, und ein zweiter Produktstrom ein Gemisch enthaltend Oxymethylenether OME₁₋₂, Wasser und Methanol umfasst, wobei man aus dem Produktstrom enthaltend OME₁₋₂ und Wasser die Oxymethylenether 0ME₁ und OME₂ durch das oben beschriebene Trennungsverfahren adsorptiv von Wasser trennt, wobei 0ME₁ und OME₂ optional der Reaktionsmischung wieder zugeführt werden.

Für die weitere Beschreibung des Trennverfahrens wird auf die vorstehende Beschreibung Bezug genommen.

Das Rohprodukt kann Oligomere der Oxymethylenether verschiedener Kettenlänge umfassen, die abhängig von den gewählten Reaktionsbedingungen in unterschiedlicher Konzentration gebildet werden. Es ist günstig, dieses zunächst destillativ in verschiedene Fraktionen aufzutrennen. Zwar lassen sich grundsätzlich alle Oxymethylenether adsorbieren, da die destillative Abtrennung der höheren Oxymethylenether ab OME₃ aus der Produktmischung jedoch gut erzielbar ist, kann hierdurch eine Vorreinigung über bekannte und etablierte destillative Schritte einfach und kostengünstig erzielt werden. Die Oxymethylenether werden daher zunächst destillativ aufgetrennt, so dass eine Fraktion enthaltend OME₃₋₅ sowie ein 0ME₁ und OME₂ und Wasser enthaltendes Gemisch getrennt voneinander vorliegen. Da OME₂ und Wasser ein Azeotrop bilden, ist Wasser in einer solchen destillativ erhaltenen Fraktion enthalten. Eine dritte Fraktion kann höhere Polyoxymethylendimethylether OME6+ umfassen. OME₁ und OME₂ werden anschließend adsorptiv mit Hilfe von Aktivkohle oder hypervernetzter Polymere vom Wasser getrennt.

Beispielsweise kann das Herstellungsverfahren ein Syntheseverfahren für Methylal und/oder OME₂ sein. Hierbei können OME₁ und OME₂ gemeinsam adsorptiv von Wasser abtrennt werden. Die Desorption kann mittels Methanol erfolgen, wodurch eine Mischung von OME₁ und OME₂ erhalten wird. Bei Verwendung eines Desorptionsmittels mit einem höheren Siedepunkt bei Atmosphärendruck, vorzugsweise > 120 °C, können Methylal und OME₂ in einem nachfolgenden Destillationsschritt getrennt werden und einzeln wasserfrei erhalten werden. In vorteilhafter Weise ist das Trennungsverfahren auch im Rahmen bekannter Herstellungsverfahren für Methylal verwendbar.

Insbesondere kann das Trennungsverfahren in vorteilhafter Weise in einem kontinuierlichen Herstellungsprozess für Oxymethylenether OME₃₋₅ verwendet werden. Hierbei werden die Oxymethylenether OME₁ und OME₂ adsorptiv von Wasser getrennt, wobei vorzugsweise Methanol für die Desorption verwendet wird, und OME₁ und OME₂ in Methanol der Reaktionsmischung wieder zugeführt werden.

In Ausführungsformen kann die destillative Trennung des Rohproduktes in einem zweischrittigen Prozess über zwei Destillationskolonnen durchgeführt werden, wobei
- über eine erste Destillation das Rohprodukt in zwei verschiede Produktströme aufgetrennt wird, wobei ein erster Produktstrom eine Mischung an Oxymethylendimethylethern OME₁₋₅ umfasst und der zweite Produktstrom Polyoxymethylendimethylether OME₆₊ umfasst und der Reaktionsmischung wieder zugeführt wird, und
- über eine zweite Destillation, wobei der erste Produktstrom umfassend die Oxymethylendimethylether OME₁₋₅ erneut in zwei verschiedene Produktströme aufgetrennt wird, wobei einer dieser Produktströme eine Mischung der Oxymethylendimethylether OME₃₋₅ umfasst, und der andere eine Produktmischung enthaltend Wasser, Methanol und OME₁₋₂ umfasst.

Somit können weiter Herstellungsverfahren für Oxymethylenether zur Verfügung gestellt werden, die eine energetisch günstige Herstellung der Oxymethylendimethylether OME₃₋₅ erlauben und eine Synthese im technischen Maßstab sowie eine damit verbundene gute Verfügbarkeit und Kommerzialisierung der Produkte zur Verfügung stellen können. Dies ist für eine kommerzielle Synthese von Methylal, das in Dieselgemischen selbst ohne Autoabgaskatalysator die EURO5 Norm erfüllt, vorteilhaft, sowie insbesondere für die Oxymethylendimethylether OME₃₋₅, die als alternative synthetische Kraftstoffe diskutiert werden.

Beispiele und Figuren, die der Veranschaulichung der vorliegenden Erfindung dienen, sind nachstehend angegeben.

Hierbei zeigt:
- Figur 1: die kontinuierliche Herstellung und Abtrennung der Oxymethylenether OME₃₋₅ gemäß einem Ausführungsbeispiel der Erfindung.
- Figur 2: die Kapazitäten sphärischer Aktivkohlen und eines hypervernetzten Homo-Polymers für OME₁ und OME₂.
- Figur 3: die Kapazitäten extrudierter und pulverförmiger Aktivkohlen für OME₁ und OME₂.
- Figur 4: die Kapazitäten verschiedener hypervernetzter Homo-Polymere und Co-Polymere für OME₂.

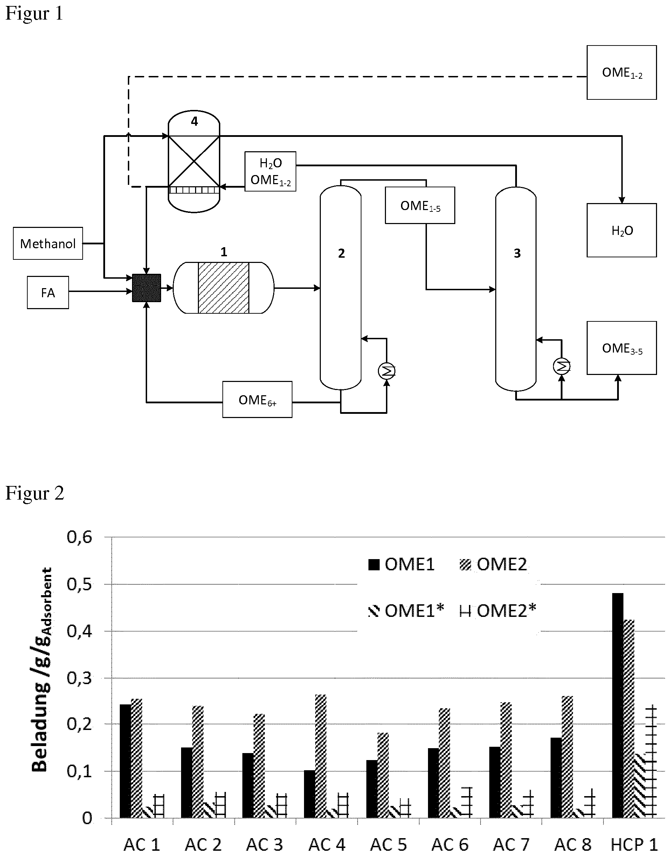

Die Figur 1 zeigt, dass eine Reaktionsmischung enthaltend Formaldehyd und Methanol in einem Reaktor 1 umgesetzt wird. Das gebildete Rohprodukt umfasst eine Mischung unterschiedlicher oligomerer Oxymethylenether, abhängig von der Reaktionsführung zumeist OME₁₋₇. Das Rohprodukt wird zunächst mittels einer zweistufigen Destillation über zwei Destillationskolonnen 2 und 3 destillativ aufgetrennt. In einer ersten Destillationskolonne 2 wird das Rohprodukt in zwei verschiedene Produktströme aufgetrennt. Ein Produktstrom der ersten Destillation enthält eine Mischung der Oxymethylenether OME₁₋₅. Dieser wird einer zweiten Destillationskolonne 3 zugeführt. Ein weiterer Produktstrom enthält aus der Umsetzung weiterhin gebildete längerkettige Polyoxymethylendimethylether OME₆₊ und wird der Reaktionsmischung wieder zugeführt.

In einer zweiten Destillationskolonne 3, die mit einem Druck betrieben wird, der höher als der Druck in der ersten Destillationskolonne 2 ist, wird der erste Produktstrom umfassend die Oxymethylenether OME₁₋₅ erneut destillativ aufgetrennt. Ein Produktstrom der zweiten Destillation enthält die gewünschten Endprodukte OME₃₋₅, die destillativ gut abgetrennt werden können. Die kleinen Oxymethylenether OME₁ und OME₂ verbleiben in einem Gemisch mit Wasser, wobei das Gemisch weitere Stoffe enthalten kann wie Methanol oder Hemiacetale.

Dieses Gemisch wird über einen Adsorber 4 durch Adsorption an Aktivkohle oder hypervernetzte Polymere (HCPs) von Wasser getrennt. Die abgetrennten Oxymethylenether OME₁ und OME₂ werden vorzugsweise mit Methanol desorbiert und können der Reaktionsmischung wieder zugeführt und in dem kontinuierlichen Verfahren somit erneut für die Herstellung der Oxymethylenether OME₃₋₅ genutzt werden. Alternativ können die abgetrennten Oxymethylenether OME₁ und OME₂ aus dem Prozess entnommen werden.

### Beispiel 1

### Herstellung von hypervernetzten Polymeren

Ein hypervernetztes Homo-Polymer wurde durch Polymerisation von 4,4-bis(chloromethyl)-1,10-biphenyl (BCMBP) hergestellt. Die Synthese erfolgte mittels Fidel-Craft Alkylierung. Dazu wurden 2 g BCMBP (Sigma-Aldrich) in 20 ml Dichlorethan (Sigma-Aldrich) unter Stickstoffatmosphäre gelöst. Unter Zugabe von 1,4 g FeCl₃ (Sigma-Aldrich) gelöst in 15 ml Dichlorethan wurde dann unter Stickstoffatmosphäre bei 80 °C die Polymerisation durchgeführt. Das erhaltene Polymer wurde für 20 Stunden in einem Soxhlet mit Methanol gewaschen. Anschließend wurde das Polymer für 24 h bei 60 °C getrocknet.

Hypervernetzte Co-Polymere wurden hergestellt, indem jeweils 2 g der jeweiligen Monomere in äquimolarer Menge oder in einem molaren Verhältnis von 3:1 eingesetzt wurden. Hierbei wurden hypervernetzte Co-Polymere aus Mischungen der folgenden Monomere hergestellt: 4,4-bis(chloromethyl)-1,10-biphenyl (BCMBP) und Triphenylamin (TPA), Triphenylbenzol (TPB), 1,4-Dichloroxylen (p-DCX) oder 2,5-Dichloro-p-xylen (2,5-p-DCX); sowie 1,4-Dichloroxylen (p-DCX) und Triphenylamin (TPA), Dibenzofuran (DBF), Dibenzothiophen (DBT), Triphenylbenzol (TPB), 1,4-Dichloroxylen (p-DCX) oder 2,5-Dichloro-p-xylen (2,5-p-DCX); sowie 1,2-Dichloroxylen (o-DCX) und Triphenylamin (TPA) oder Dibenzofuran (DBF).

Die spezifische Oberfläche der erhaltenen hypervernetzten Polymere wurde durch Gasadsorption nach dem Brunauer-Emmett-Teller (BET)-Verfahren unter Verwendung eines QUADRASORB IS (Quantachrome Instruments) mittels Stickstoff-Adsorption bestimmt. Hieraus wurde das Porenvolumen berechnet. Weiter wurde die Wasseradsorption mittels eines Autosorb iQ₂ (Quantachrome Instruments) bestimmt und die Polarität wurde aus dem Quotienten des Porenvolumens der Wasseradsorption und der BET-Oberfläche bestimmt.

Die folgende Tabelle 1 fasst die hergestellten hypervernetzten Polymere und deren bestimmte Oberflächeneigenschaften zusammen:

**Tabelle 1: Oberflächeneigenschaften der hypervernetzten Polymere**

| HCPs | | | Oberflächen eigenschaften | | | |
|---|---|---|---|---|---|---|
| | Monomere | | spezifische Oberfläche, m²g⁻¹ | Porenvolumen BET/ cm³g⁻¹ | Wasseradsorption cm³g⁻¹ | Quotient aus Porenvolumen der Wasseradsorption und BET |
| HCP 1 | BCMBP | 1 | 2090 | 2,0690 | 0,1403 | 0,0678 |
| HCP 2 | BCMBP + TPA | 3:1 | 1229 | 0,9956 | 0,1094 | 0,1099 |
| HCP 3 | BCMBP + TPB | 3:1 | 1293 | 1,1150 | 0,0958 | 0,0859 |
| HCP 4 | BCMBP + pDCX | 1:1 | 1537 | 1,6280 | 0,1304 | 0,08009 |
| HCP 5 | BCMBP + 2,5-pDCX | 1:1 | 908 | 0,8008 | n.b. | n.b. |
| HCP 6 | p-DCX + TPA | 3:1 | 951 | 0,7821 | n.b. | n.b. |
| HCP 7 | p-DCX + DBF | 3:1 | 1142 | 0,8453 | n.b. | n.b. |
| HCP 8 | p-DCX + DBT | 3:1 | 902 | 0,5235 | n.b. | n.b. |
| HCP 9 | p-DCX + TPB | 3:1 | 1345 | 1,2430 | n.b. | n.b. |
| HCP 10 | p-DCX + o-DCX | 1:1 | 1254 | 1,1560 | n.b. | n.b. |
| HCP 11 | p-DCX + 2,5-pDCX | 1:1 | 669 | 0,5609 | n.b. | n.b. |
| HCP 12 | o-DCX + TPA | 3:1 | 582 | 0,4200 | n.b. | n.b. |
| HCP 13 | o-DCX + DBF | 3:1 | 1045 | 0,8027 | n.b. | n.b. |

Hierbei bedeutet n.b. nicht bestimmt. Wie man der Tabelle 1 entnimmt, zeigten die hergestellten hypervernetzten Polymere eine große spezifische Oberfläche und eine sehr unpolare Oberfläche.

### Beispiel 2

Bestimmung der Adsorption von OME₁ und OME₂ an sphärischer Aktivkohle und einem hypervernetzten Homo-Polymer

In Batch-Adsorptionsexperimenten wurde die Adsorptionskapazität, die Menge an OME₁ oder OME₂, die pro Menge Adsorbens aufgenommen wurde, bestimmt. Die Oxymethylenether 0ME₁ und OME₂ wurden von der Wacker Chemie AG synthetisiert und aufgereinigt bezogen.

Die Adsorption wurde jeweils für eine hohe wie für eine niedrige Ausgangskonzentration an Oxymethylenether von 10 Gew.-% und 1 Gew.-%, bezogen auf das Gesamtgewicht eines Gemischs von Oxymethylenether, Methanol und Wasser, bestimmt. Hierzu wurden Mischungen aus Methanol:Wasser:OME₁ und Methanol:Wasser:OME₂ mit einem Gewichtsverhältnis von 4,5:4,5:1 (Gewichtsanteile) sowie mit einem Gewichtsverhältnis von 49,5:49,5:1 (Gewichtsanteile) hergestellt.

Als Adsorbentien wurden sphärische Aktivkohlen AC1 bis AC8 erhältlich bei der Blücher GmbH sowie das hypervernetzte Homo-Polymer von 4,4-bis(chloromethyl)-1,10-biphenyl (HCP 1) aus Beispiel 1 verwendet. Die folgende Tabelle 2 fasst die Eigenschaften der sphärischen Aktivkohlen zusammen:

**Tabelle 2: sphärische Aktivkohlen (Blücher GmbH)**

| | Struktureigenschaften | |
|---|---|---|
| Aktivkohle | spezifische Oberfläche, m²g⁻¹ | mittlere Partikelgröße |
| AC1: Art. Nr 100772 | 1425 | 0,45 mm |
| AC2: Art. Nr 100042 | | 0,2 mm |
| AC3: Art. Nr 101373 | | <0,63 l >0,2 mm |
| AC4: Art. Nr 105962 | 1274 | 0,63 mm |
| AC5: Art. Nr 104113 | | <0,63 l >0,2 mm |
| AC6: Art. Nr 100050 | 1889 | 0,2 mm |
| AC7: Art. Nr 101406 | 1771 | <0,63 l >0,2 mm |
| AC8: Art. Nr. 101408 | 1915 | <0,63 l >0,2 mm |

In einem Glasgefäß (Glasveil) wurden 500 mg der jeweiligen Aktivkohle oder 100 mg des hypervernetzten Polymers abgewogen und 4 ml der hergestellten Mischungen hinzugegeben. Die Batch-Adsorptionsexperimente wurden in einem Wasserschüttelbad (GFL) durchgeführt, wobei die Mischung mit den jeweiligen Adsorbentien Aktivkohle und HCP für 1 Stunde bei einer Temperatur von 20 °C geschüttelt wurden, so dass sich ein Gleichgewichtszustand der Beladung einstellen konnte. Anschließend wurde der Feststoff abgefiltert und getrocknet. Die Flüssigkeit sowie die Substratmischung wurden mittels Gaschromatographie (Trace5, ThermoFischer) analysiert. Als interner Standard wurde n-Pentanol verwendet. Aus der Konzentrationsdifferenz zwischen Substratmischung und abgefilterter Probe wurde die adsorbierte Menge an OME₁ oder OME₂ bestimmt. Die Beladung ergibt sich dann aus dem Quotienten der adsorbierten Menge an OME₁ oder OME₂ und der Masse des Adsorbens.

Das Balkendiagramm der Figur 2 zeigt die Kapazitäten der getesteten sphärischen Aktivkohlen und des hypervernetzten Homo-Polymers HCP 1 jeweils für OME₁ und OME₂ in Konzentrationen von 10 Gew.-% und 1 Gew.-%, wobei die Konzentrationen von 1 Gew.-% OME₁ oder OME₂ mit einem * gekennzeichnet sind. Wie man der Figur 2 entnimmt, zeigten die verwendeten Aktivkohlen eine gute Kapazität für OME₁ und OME₂, wobei die Kapazität für OME₂, sowohl bei hoher wie auch niedriger Konzentration jeweils höher war. Weiterhin zeigte das hypervernetzte Homo-Polymer (HCP/BCMBP) eine Kapazität, die die Kapazität der Aktivkohlen übertraf. Dies zeigt, dass OME₁ und OME₂ an sphärische Aktivkohle und hypervernetzte Polymere mit hoher Kapazität adsorbiert werden und aus einem wässrigen Gemisch von Wasser getrennt werden können.

### Beispiel 3

Bestimmung der Adsorption von OME₁ und OME₂ an extrudierter und pulverförmiger Aktivkohle

Die Bestimmung der Adsorptionskapazität von verschiedenen pulverförmigen Aktivkohlen für OME₁ und OME₂ (Wacker Chemie AG) wurde wie in Beispiel 2 beschrieben in Batch-Adsorptionsexperimenten bestimmt. Die Adsorption wurde jeweils für eine Ausgangskonzentration an Oxymethylenether von 10 Gew.-%, bezogen auf das Gesamtgewicht eines Gemischs von Oxymethylenether, Methanol und Wasser, bestimmt. Als Adsorbentien wurden extrudierte zylindrische Aktivkohlen AC9 = Norit® RB3 630168 (mittlere Länge 3 mm), AC10 = Norit® ROX 0.8 500159 (mittlere Länge 0,8 mm), AC11 = Norit® RX 3 Extra 580092 (mittlere Länge 3 mm), sowie pulverförmige Aktivkohlen AC12 = Norit® Carbot A Supra EUR 8024.3, AC13 = Norit® A Supra Code: 134342500, AC14 = Norit® SX-1G 8050-0 und AC15 = Norit® DARCO G60 8010-2, alle erhältlich bei Cabot Corporation, USA verwendet. Wie in Beispiel 2 beschrieben wurden in einem Glasgefäß 500 mg der jeweiligen Aktivkohle mit 4 ml der Oxymethylenether-Mischungen vermischt und die Beladung bestimmt.

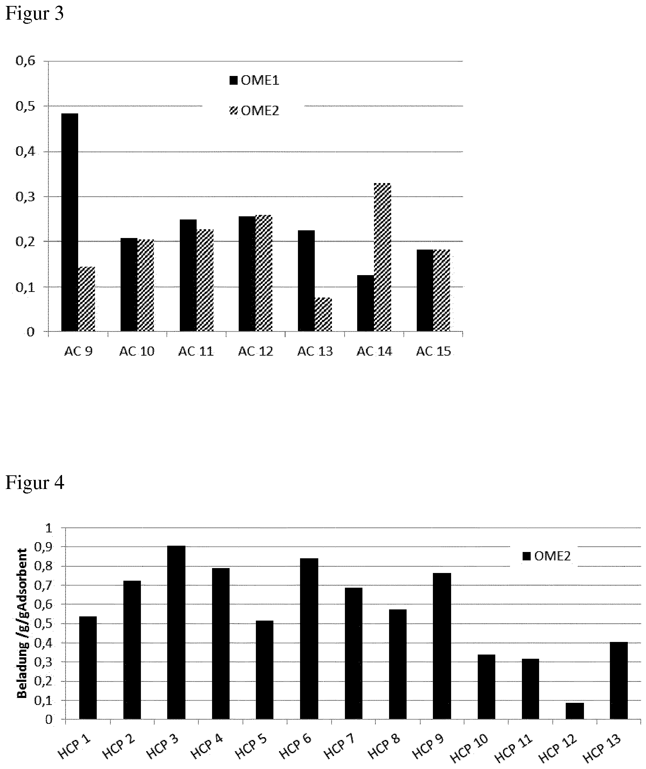

Das Balkendiagramm der Figur 3 zeigt die Kapazitäten der getesteten extrudierten und pulverförmigen Aktivkohlen AC 9 bis Ac15 für Gemische enthaltend 10 Gew.-% OME₁ oder OME₂. Wie man der Figur 3 entnimmt, zeigten auch die extrudierten und pulverförmigen Aktivkohlen eine gute Kapazität für OME₁ und OME₂. Dies zeigt, dass OME₁ und OME₂ auch an diese Aktivkohlen mit hoher Kapazität adsorbiert werden und mit diesem Adsorbens aus einem wässrigen Gemisch getrennt werden können.

### Beispiel 4

Bestimmung der Adsorption von OME₂ an hypervernetzten Homo-Polymeren und CoPolymeren

Die Bestimmung der Adsorptionskapazität für OME₂ wurde für die gemäß Beispiel 1 hergestellten hypervernetzten Co-Polymere HCP 2 bis HCP 14 in Batch-Adsorptionsexperimenten wie in Beispiel 2 beschrieben bestimmt. Die Adsorption wurde jeweils für eine Ausgangskonzentration an 10 Gew.-% OME₂ (Wacker Chemie AG), bezogen auf das Gesamtgewicht eines Gemischs von OME₂, Methanol und Wasser, bestimmt. Wie in Beispiel 2 beschrieben wurden in einem Glasgefäß 100 mg des jeweiligen hypervernetzten Polymers mit 4 ml der Oxymethylenether-Mischungen vermischt und die Beladung bestimmt.

Das Balkendiagramm der Figur 4 zeigt die Kapazitäten der hypervernetzten Homo-Polymere sowie Co-Polymere für OME₂. Wie man der Figur 4 entnimmt, zeigten alle verwendeten hypervernetzten Co-Polymere eine sehr gute Kapazität für OME₂, wobei diese bis auf das Co-Polymer o-DCX/TPA die Kapazität der Aktivkohlen überstieg.

Die Ergebnisse zeigen insgesamt, dass Oxymethylenether an Aktivkohle und hypervernetzte Homo-Polymere sowie Co-Polymere adsorbiert werden und hierdurch aus wasserhaltigen Gemischen abgetrennt werden können.

### Beispiel 5

Bestimmung der Adsorption von OME₁ an hypervernetztem Co-Polymer in Form granulatähnlicher Partikel

Ein hypervernetztes Polymer wurde durch Polymerisation von 4,4-bis(chloromethyl)-l,10-biphenyl (BCMBP) und Triphenylbenzol (TPB) hergestellt. Die Synthese erfolgte mittels Fidel-Craft Alkylierung. Dazu wurden 2,5 g BCMBP (Sigma-Aldrich) und 1 g TPB in 40 ml Dichlorethan (Sigma-Aldrich) unter Stickstoffatmosphäre gelöst. Unter Zugabe von 4 g FeCl₃ (Sigma-Aldrich) gelöst in 40 ml Dichlorethan wurde dann unter strömendem Stickstoff bei 80 °C die Polymerisation durchgeführt. Das erhaltene Polymer wurde für 20 Stunden in einem Soxhlet mit Methanol gewaschen. Anschließend wurde das Polymer für 24 h bei 60 °C getrocknet. Das erhaltene Polymer wies eine Korngröße von 600 µm - 8000 µm auf.

Die Adsorption wurde anschließend in einem Adsorber durchgeführt. Der Adsorber umfasste ein Rohr mit einem Durchmesser von 6 mm, einer Fritte, die das Polymer zurückhält, und einer HPLC-Pumpe, um die Adsorptionslösung zu pumpen. Es wurden 0,5 g des Polymers in das Rohr eingefüllt und mit einem Volumenstrom von 0.5 ml/min wurde die Adsorptionslösung aus 10 wt% OME₁ und 90 wt% Wasser durch die Adsorptionsschüttung gepumpt. Die nachfolgende Tabelle 3 zeigt die Adsorption bei verschiedenen Adsorptionstemperaturen:

**Tabelle 3: Adsorption bei verschiedenen Adsorptionstemperaturen**

| Adsorptionstemperatur | Beladung g*g⁻¹ |
|---|---|
| 25°C | 0,58 |
| 40°C | 0,65 |
| 60°C | 0,66 |
| 80°C | 0,44 |

Dies zeigt, dass das partikuläre Copolymer OME₁ mit hoher Kapazität adsorbierte. Hierbei wurde im beobachteten Bereich eine gute Adsorption erzielt, insbesondere zwischen 25°C und 60°C.

### Beispiel 6

### Bestimmung der Desorption

Die mit OME₁ beladenen Polymerpartikel aus Beispiel 5 wurden in Ethanol bei verschiedenen Temperaturen desorbiert. Die nachfolgende Tabelle 4 zeigt die erhaltenen scheinbaren Desorptionsentladungen bei den verwendeten Temperaturen:

**Tabelle 4: Desorption bei verschiedenen Desorptionstemperaturen**

| Desorptionstemperatur | Entladung 0ME₁ bei Desorptions-Temperatur g*g⁻¹ | Kumulierte Summe der Entladung |
|---|---|---|
| 40°C | 0,4116 | 0,539175 |
| 60°C | 0,2544 | 0,872413 |
| 80°C | 0,0709 | 0,965351 |
| 100°C | 0,0206 | 0,992361 |
| 120°C | 0,0058 | 1 |
| total | 0,7634 | 1 |

Die Desorptionsentladung entspricht hierbei der enthaltenen Gesamtmasse an OME₁ welches desorbiert wurde bezogen auf das eingewogene Gewicht des Polymergranulates. Die Massen wurden jeweils mittels Gas-Chromatographie (GC) bestimmt. Weiterhin wird in Tabelle 4 die kumulierte Summe der Entladung angegeben. Diese entspricht der prozentualen Entladung, welche bei der entsprechenden Desorptionstemperatur erreicht wurde.

Die Ergebnisse der Tabelle 4 zeigen, dass bereits bei 40°C ca. 54 wt% der adsorbierten Masse desorbiert wurden. Für eine volle Entladung (Desorption) des Polymers wurde die Temperatur weiter erhöht bis bei 100°C eine volle Entladung erzielt wurde.

### Beispiel 7

### Bestimmung der Wiederverwendbarkeit der Polymere

Um eine Wiederverwendbarkeit der Polymere zu zeigen, wurde das gemäß Beispiel 5 hergestellte Polymer verwendet und der Zyklus der in Beispiel 5 und 6 beschriebenen Adsorption und Desorption mehrere Male wiederholt. Dazu wurde jeweils bei 25°C adsorbiert und bei 80°C desorbiert. Die Tabelle 5 zeigt die Beladung bezogen auf die desorbierte Masse für den jeweiligen Zyklus.

**Tabelle 5: Zyklische Untersuchung des Polymers**

| Zyklus | Beladung des jeweiligen Zyklus g*g⁻¹ |
|---|---|
| 1 | 0,2735 |
| 2 | 0,7222 |
| 3 | 0,3961 |
| 4 | 0,4154 |
| 5 | 0,4116 |

Wie man der Tabelle 5 entnimmt, zeigte das Polymer ab dem vierten Zyklus eine konstante Beladung von ca. 0,4 g*g⁻¹. Dies zeigt insgesamt eine gute Wiederverwendbarkeit des Polymers bezogen auf die Kapazität von OME₁.

## Patentansprüche

1. Verfahren zur Trennung von Oxymethylenethern aus einem diese enthaltenden Gemisch, **dadurch gekennzeichnet, dass** man die Oxymethylenether durch Adsorption an Adsorbentien ausgewählt aus der Gruppe umfassend Aktivkohle und/oder hypervernetzte Polymere abtrennt.

2. Verfahren nach Anspruch 1, **dadurch gekennzeichnet, dass** das Gemisch Wasser enthält.

3. Verfahren nach Anspruch 1 oder 2, **dadurch gekennzeichnet, dass** die Oxymethylenether ausgewählt sind aus der Gruppe umfassend Methylal und/oder 2,4,6-Trioxaheptan.

4. Verfahren nach einem der vorhergehenden Ansprüche, **dadurch gekennzeichnet, dass** man die Adsorption bei einer Temperatur im Bereich von ≥ 0 °C bis ≤ 120 °C, bevorzugt im Bereich von ≥ 10°C bis ≤ 90°C, vorzugsweise bei einer Temperatur im Bereich von ≥ 20°C bis ≤ 40°C durchführt.

5. Verfahren nach einem der vorhergehenden Ansprüche, **dadurch gekennzeichnet, dass** man eine nachfolgende Desorption bei einer Temperatur im Bereich von ≥ 0 °C bis ≤ 250 °C, bevorzugt im Bereich von ≥ 20°C bis ≤ 200°C, vorzugsweise bei einer Temperatur im Bereich von ≥ 60°C bis ≤ 80°C, durchführt.

6. Verfahren nach einem der vorhergehenden Ansprüche, **dadurch gekennzeichnet, dass** man die nachfolgende Desorption mittels eines Lösungsmittels ausgewählt aus der Gruppe umfassend Alkohole, Ether, Aldehyde, Ketone und/oder deren Mischungen durchführt, wobei das Lösungsmittel vorzugsweise Methanol ist.

7. Verfahren nach einem der vorhergehenden Ansprüche, **dadurch gekennzeichnet, dass** die hypervernetzten Polymere ausgebildet sind aus Monomeren ausgewählt aus der Gruppe umfassend 4,4-bis(chloromethyl)-1,10-biphenyl, 1,2-Dichloroxylen, 1,3-Dichloroxylen, 1,4-Dichloroxylen, 2,5-Dichloro-p-xylen, Triphenylamin, Dibenzofuran, Dibenzothiophen, Triphenylbenzol und/oder Fluoren.

8. Verfahren nach einem der vorhergehenden Ansprüche, **dadurch gekennzeichnet, dass** die spezifische Oberfläche der Aktivkohle im Bereich von ≥ 600 m²/g bis ≤ 3000 m²/g liegt, und/oder die spezifische Oberfläche der hypervernetzte Polymere im Bereich von ≥ 800 m²/g bis ≤ 3000 m²/g liegt, wobei die spezifische Oberfläche nach dem BET-Verfahren bestimmt wird.

9. Verfahren nach einem der vorhergehenden Ansprüche, **dadurch gekennzeichnet, dass** man Aktivkohle oder hypervernetzte Polymere in Form sphärischer Partikel oder als Pulver verwendet, wobei die sphärischen Partikel vorzugsweise einen mittleren Durchmesser im Bereich von ≥ 0,005 mm bis ≤ 200 mm, bevorzugt im Bereich von ≥ 0,05 mm bis ≤ 20 mm, aufweisen.

10. Kontinuierliches Verfahren zur Herstellung und Abtrennung von Oxymethylenethern durch Umsetzen einer Reaktionsmischung enthaltend Formaldehyd und Methanol, wobei man das gebildete Rohprodukt destillativ in verschiedene Produktströme trennt, wobei ein erster Produktstrom eine Mischung an Oxymethylendimethylethern OME₃₋₅ umfasst, und ein zweiter Produktstrom ein Gemisch enthaltend Oxymethylenether OME₁₋₂, Wasser und Methanol umfasst, **dadurch gekennzeichnet, dass** man aus dem Produktstrom enthaltend OME₁₋₂ und Wasser die Oxymethylenether OME₁ und OME₂ durch das Verfahren gemäß einem der Ansprüche 1 bis 9 adsorptiv von Wasser trennt, wobei OME₁ und OME₂ optional der Reaktionsmischung wieder zugeführt werden.

## Claims

1. Process for the separation of oxymethylene ethers from a mixture containing these, **characterized in that** the oxymethylene ethers are separated off by adsorption on adsorbents selected from the group comprising activated carbon and/or hypercrosslinked polymers.

2. Process according to Claim 1, **characterized in that** the mixture contains water.

3. Process according to Claim 1 or 2, **characterized in that** the oxymethylene ethers are selected from the group comprising methylal and/or 2,4,6-trioxaheptane.

4. Process according to any of the preceding claims, **characterized in that** the adsorption is conducted at a temperature in the range from ≥ 0°C to ≤ 120°C, preferably in the range from ≥ 10°C to ≤ 90°C, and with preference at a temperature in the range from ≥ 20°C to ≤ 40°C.

5. Process according to any of the preceding claims, **characterized in that** a subsequent desorption is conducted at a temperature in the range from ≥ 0°C to ≤ 250°C, preferably in the range from ≥ 20°C to ≤ 200°C, and with preference at a temperature in the range from ≥ 60°C to ≤ 80°C.

6. Process according to any of the preceding claims, **characterized in that** the subsequent desorption is conducted using a solvent selected from the group comprising alcohols, ethers, aldehydes, ketones and/or mixtures thereof, the solvent preferably being methanol.

7. Process according to any of the preceding claims, **characterized in that** the hypercrosslinked polymers are formed from monomers selected from the group comprising 4,4-bis(chloromethyl)-1,10-biphenyl, 1,2-dichloroxylene, 1,3-dichloroxylene, 1,4-dichloroxylene, 2,5-dichloro-p-xylene, triphenylamine, dibenzofuran, dibenzothiophene, triphenylbenzene and/or fluorene.

8. Process according to any of the preceding claims, **characterized in that** the specific surface area of the activated carbon is in the range from ≥ 600 m²/g to ≤ 3000 m²/g, and/or the specific surface area of the hypercrosslinked polymers is in the range from ≥ 800 m²/g to ≤ 3000 m²/g, the specific surface area being determined by the BET method.

9. Process according to any of the preceding claims, **characterized in that** activated carbon or hypercrosslinked polymers are used in the form of spherical particles or as powder, wherein the spherical particles preferably have a mean diameter in the range from ≥ 0.005 mm to ≤ 200 mm, preferably in the range from ≥ 0.05 mm to ≤ 20 mm.

10. Continuous process for the preparation and separation of oxymethylene ethers by converting a reaction mixture containing formaldehyde and methanol, wherein the crude product formed is separated into various product streams by distillation, wherein a first product stream comprises a mixture of oxymethylene dimethyl ethers OME₃₋₅, and a second product stream comprises a mixture containing oxymethylene ethers OME₁₋₂, water and methanol, **characterized in that** the oxymethylene ethers OME₁ and OME₂ are separated, by adsorption from water, out of the product stream containing OME₁₋₂ and water by the process according to any of Claims 1 to 9, wherein OME₁ and OME₂ are optionally resupplied to the reaction mixture.

## Revendications

1. Procédé de séparation d'oxyméthylène éthers à partir d'un mélange les contenant, **caractérisé en ce qu'**on sépare les oxyméthylène éthers par adsorption sur des adsorbants choisis dans le groupe comprenant le charbon actif et/ou les polymères hyper-réticulés.

2. Procédé selon la revendication 1, **caractérisé en ce que** le mélange contient de l'eau.

3. Procédé selon la revendication 1 ou 2, **caractérisé en ce que** les oxyméthylène éthers sont choisis dans le groupe comprenant le méthylal et/ou le 2,4,6-trioxaheptane.

4. Procédé selon l'une des revendications précédentes, **caractérisé en ce qu'**on met en œuvre l'adsorption à une température dans la plage de ≥ 0 °C à ≤ 120 °C, de préférence dans la plage de ≥ 10 °C à ≤ 90 °C, préférentiellement à une température dans la plage de ≥ 20 °C à ≤ 40 °C.

5. Procédé selon l'une des revendications précédentes, **caractérisé en ce qu'**on met en œuvre une désorption subséquente à une température dans la plage de ≥ 0 °C à ≤ 250 °C, de préférence dans la plage de ≥ 20 °C à ≤ 200 °C, de préférence à une température dans la plage de ≥ 60 °C à ≤ 80 °C.

6. Procédé selon l'une des revendications précédentes, **caractérisé en ce que** la désorption subséquente est mise en œuvre à l'aide d'un solvant choisi dans le groupe comprenant les alcools, les éthers, les aldéhydes, les cétones et/ou les mélanges de ceux-ci, le solvant étant de préférence le méthanol.

7. Procédé selon l'une des revendications précédentes, **caractérisé en ce que** les polymères hyper-réticulés sont formés à partir de monomères choisis dans le groupe comprenant le 4,4-bis(chlorométhyl)-1,10-biphényle, le 1,2-dichloroxylène, le 1,3-dichloroxylène, le 1,4-dichloroxylène, le 2,5-dichloro-p-xylène, la triphénylamine, le dibenzofurane, le dibenzothiophène, le triphénylbenzène et/ou le fluorène.

8. Procédé selon l'une des revendications précédentes, **caractérisé en ce que** l'aire spécifique du charbon actif est comprise dans la plage de ≥ 600 m²/g à ≤ 3 000 m²/g, et/ou l'aire spécifique des polymères hyper-réticulés est comprise dans la plage de ≥ 800 m²/g à ≤ 3 000 m²/g, l'aire spécifique étant déterminée par le procédé BET.

9. Procédé selon l'une des revendications précédentes, **caractérisé en ce qu'**on utilise le charbon actif ou les polymères hyper-réticulés sous forme de particules sphériques ou sous forme d'une poudre, les particules sphériques présentant de préférence un diamètre moyen dans la plage de ≥ 0,005 mm à ≤ 200 mm, de préférence dans la plage de ≥ 0,05 mm à ≤ 20 mm.

10. Procédé continu de fabrication et de séparation d'oxyméthylène éthers par réaction d'un mélange réactionnel contenant du formaldéhyde et du méthanol, dans lequel on sépare par distillation le produit brut formé en différents courants de produit, un premier courant de produit comprenant un mélange d'oxyméthylène diméthyléthers OME₃₋₅, et un second courant de produit comprenant un mélange contenant des oxyméthylène éthers OME₁₋₂, de l'eau et du méthanol, **caractérisé en ce qu'**on sépare du courant de produit contenant l'OME₁₋₂ et de l'eau les oxyméthylène éthers OME₁ et OME₂ par le procédé selon l'une des revendications 1 à 9 par adsorption d'eau, l'OME₁ et l'OME₂ étant éventuellement renvoyés dans le mélange réactionnel.
